# EUROPÄISCHE PATENTSCHRIFT

(11) **EP 1 556 697 B1**
(45) Veröffentlichungstag und Bekanntmachung des Hinweises auf die Patenterteilung: **03.10.2007**
(21) Anmeldenummer: 03809742.4
(22) Anmeldetag: 29.10.2003
(51) Int. Cl.: G01N 33/571, G01N 33/92, G01N 33/68, C07K 14/20, C12Q 1/68

(54) **MITTEL UND VERFAHREN ZUR DIAGNOSE EINER TREPONEMAINFEKTION**
MEANS AND METHODS FOR DIAGNOSING A TREPONEMA INFECTION
AGENTS ET METHODES DE DIAGNOSTIC D'UNE INFECTION A TREPONEMES

(30) Priorität: 29.10.2002 DE 10250368; 21.11.2002 WO PCT/EP02/13088
(43) Veröffentlichungstag der Anmeldung: 27.07.2005
(73) Patentinhaber: Viramed Biotech AG, 82152 Planegg (DE)
(72) Erfinder: KINTRUP, Martin, 82131 Gauting (DE); THÜRING-NAHLER, Heike, 90522 Oberasbach (DE); KRONSTEINER, Lilly, 82152 Planegg (DE); HELBL, Vera, 80689 München (DE); ENGEL, Heinz, 80689 München (DE); FURTMAYR, Ludwig, 86989 Steingaden (DE)
(74) Vertreter: Grünecker, Kinkeldey, Stockmair & Schwanhäusser Anwaltssozietät
(86) Internationale Anmeldenummer: PCT/EP2003/012011
(87) Internationale Veröffentlichungsnummer: WO 2004/040311

(56) Entgegenhaltungen:
- WO-A-91/10138
- WO-A-97/13151
- US-A- 3 564 089
- KREBS A ET AL: "LUES, SEUCHE UND SEROLOGIE SYPHILIS UP-TO-DATE, INFECTION AND SEROLOGY" PRAXIS, HUBER, BERN, CH, Bd. 71, Nr. 46, 16. November 1982 (1982-11-16), Seiten 1807-1811, XP009019593 ISSN: 0369-8394
- MIRANDA A ET AL: "A comparison of VDRL and immunoassays developed with a recombinant TmpA antigen in the screening of anitbodies to Treponema pallidum" SERODIAGNOSIS AND IMMUNOTHERAPY IN INFECTIOUS DISEASE, ACADEMIC PRESS, LONDON, GB, Bd. 8, Nr. 3/4, 1997, Seiten 149-155, XP009019601 ISSN: 0888-0786
- YOUNG H ET AL: "A new recombinant antigen latex agglutination test (Syphilis Fast) for the rapid serological diagnosis of syphilis" INTERNATIONAL JOURNAL OF STD AND AIDS, ROYAL SOCIETY OF MEDICINE SERVICES, LONDON, GB, Bd. 9, Nr. 4, April 1998 (1998-04), Seiten 196-200, XP009019582 ISSN: 0956-4624
- PEDERSEN N S ET AL: "ELISA FOR DETECTION OF ANTIBODIES TO THE VENEREAL DISEASE RESEARCH LABORATORY VDRL ANTIGEN IN SYPHILIS" JOURNAL OF CLINICAL MICROBIOLOGY, Bd. 25, Nr. 9, 1987, Seiten 1711-1716, XP009024598 ISSN: 0095-1137
- LEFEVRE J-C ET AL: "EVALUATION OF THE CAPTIA ENZYME IMMUNOASSAYS FOR DETECTION OF IMMUNOGLOBULINS G AND M TO TREPONEMA-PALLIDUM IN SYPHILIS" JOURNAL OF CLINICAL MICROBIOLOGY, Bd. 28, Nr. 8, 1990, Seiten 1704-1707, XP009024599 ISSN: 0095-1137
- LARSEN S A ET AL: "Laboratory diagnosis and interpretation of tests for syphilis" CLINICAL MICROBIOLOGY REVIEWS, WASHINGTON, DC, US, Bd. 8, Nr. 1, 1995, Seiten 1-21, XP002259001 ISSN: 0893-8512
- ZREIN M ET AL: "Recombinant antigen-based enzyme immunoassay for screening of Treponema pallidum antibodies in blood bank routine" JOURNAL OF CLINICAL MICROBIOLOGY, WASHINGTON, DC, US, Bd. 33, Nr. 3, März 1995 (1995-03), Seiten 525-527, XP002259000 ISSN: 0095-1137

## Beschreibung

Die vorliegende Erfindung betrifft einen Träger für die Diagnostik und/oder Verlaufskontrolle einer Treponemainfektion sowie ein Diagnostikverfahren unter Einsatz des genannten Trägers. Treponemainfektionen können damit detektiert, bestätigt und im Krankheitsverlauf kontrolliert werden.

Die Klinik der Syphilis kann grundsätzlich in unterschiedlichen Stadien verlaufen, die als Primärsyphilis, Sekundärsyphilis und Tertiärsyphilis bezeichnet werden.

Ein direkter Erregemachweis ist lediglich im Primärstadium eine gute Diagnosemöglichkeit. Der Nachweis erfolgt hierbei direkt aus der Gewebeprobe und kann durch Dunkelfeldmikroskopie, direkte Immunfluoreszenz oder eine Treponema pallidum spezifische PCR durchgeführt werden.

Serologische Verfahren zum Antikörpernachweis sind in allen Krankheitsstadien gut möglich. Sie geben Aufschluss über charakteristische Antikörperkonstellationen, die Informationen über die Behandlungsbedürftigkeit geben.

Im wesentlichen spielen dabei anti-Treponema pallidum-spezifische Antikörper und Antikörper gegen Cardiolipin eine Rolle.

Anti-Cardiolipin-Antikörper vom IgG- oder IgM-Typ können bei Patienten mit Treponema Infektionen vorkommen und können mit dem CMT - Test (Cardiolipin - Mikroflockungs - Test, englisch VDRL Test: Veneral Disease Research Laboratories Test) oder dem RPR - Test (Rapid Plasma Reagin-Test) oder dem Cardiolipin Komplement-bindungsreaktions-Test nachgewiesen werden. Bei diesen Analysevertahren werden positive Signale beim Vorhandensein von anti-lipoidalen Antikörpern in der Probe beobachtet. Der VDRL Test kann bei entsprechenden Probenverdünnungen quantitativ durchgeführt werden, so dass ein Titer, bei dem eine deutliche Flockung gerade noch auftritt, als positiv gewertet wird. Der Test eignet sich gut zur Verlaufskontrolle und Therapiekontrolle, kann aber auch unspezifisch reagieren. Kreuzreaktionen treten unter anderem bei Autoimmunerkrankungen auf, wie zum Beispiel beim Anti-Phospholipid-Syndrom oder beim Systemischen Lupus Erythematodes.

Anti-Treponema-pallidum-spezifische Antikörper können gegen Proteine aus Treponema pallidum gebildet werden. Der Nachweis dieser Antikörper kann als Suchtest, als TPHA (Treponema Pallidum Hämagglutionations Test), TPPA (Treponema Pallidum Partikel Agglutionations Test) oder TPLA (Treponema Pallidum Latex Agglutinations Test) durchgeführt werden.

Die diagnostisch relevanten Proteine aus Treponema pallidum umfassen unter anderem die Proteine mit Molekulargewichten von 47 kD, 44,5 kD, 37 kD, 17 kD und 15 kD (Labor und Diagnose, Herausgeber: Lothar Thomas, Seiten 1234 ff., TH-Books Verlagsgesellschaft mbH, Frankfurt/Main, 2000; DE19536166; W08802403; Sambri et al., Clin. Diagn. Lab. Immunol. 2001 May;8(3):534-9). In unterschiedlichen marktgängigen Testen (Innolia Syphilis, Innogenetics, Belgien; Treponema Marblot, MarDx, USA; Treponema Recomblot, Mikrogen, Deutschland) werden Kombinationen aus diesen Proteinen auf festphasigen Trägermaterialien immobilisiert. Während der Inkubation der Probe mit dem beschichteten Trägermaterial können Treponema pallidum spezifische Antikörper an die Antigene binden und nachfolgend durch eine immunochemische Reaktion nachgewiesen werden. Der Nachweis dieser Treponema pallidum spezifischen Antikörper kann im Format eines ELISA (Enzyme linked immunosorbent assay) zum Beispiel als Suchtest für Syphilis eingesetzt werden oder im Format von Immunoblots als Bestätigungstest für Syphilis.

Immunoblots werden daher bei unklaren Befunden aus den Suchreaktionen, als Bestätigungstests und zur Verlaufskontrolle eingesetzt.

Zur Bewertung der Behandlungsbedürftigkeit sind vor allem Testverfahren zur Bestimmung von anti-Treponema-pallidum-spezifischen IgM-Antikörpern von Bedeutung. Typischerweise ist dieser Nachweis mit einem Tp-IgM-FTA Test (Fluoreszenz Treponema Antikörper-Absorptions-Test) oder einem Tp-IgM-Elisa oder einem Treponema pallidum IgM Immunoblot möglich.

Zur serologischen Verlaufskontrolle und Therapiekontrolle sind vornehmlich Verfahren indiziert, die eine zumindest semiquantitative Bestimmung von anti-Cardiolipin-Antikörpem beinhalten.

In US 3,564,089 wird die Kombination von Reiter Treponemen und VDRL-Antigenen in einem Test verwendet, um entsprechende Antikörper im Patientenmaterial nachzuweisen. Dieser Nachweis von Antikörpern, die gegen Antigene der Reiter-Treponemen gerichtet sind, ist hingegen nicht geeignet, um spezifisch auch diejenigen Antikörper nachzuweisen, die gegen den eigentlichen Erreger der Syphilis (Treponema pallidum) gerichtet sind.

Das Testformat der US3564089 ermöglicht keine getrennte Detektion der Antikörperreaktion mit den Reiter-Antigenen bzw. den VDRL-Antigenen. Vielmehr ist die nachgewiesene Antikörperreaktivität die Summe beider Reaktivitäten. Eine separate Detektion der verschiedenen Antikörperreaktivitäten in dem Patientenmaterial, wie z.B. einerseits die Reaktivität mit den Treponemen-spezifischen Antigenen und andererseits dem VDRL-Antigenen, hat jedoch Vorteile in der Diagnostik, wie an späterer Stelle noch ausführlich erläutert wird.

Die aus dem Stand der Technik bekannten zur serologischen Bestätigung geeigneten Nachweisverfahren erlauben keine quantitative Bestimmung der Antikörper in der Patientenprobe und keine Unterscheidung zwischen verschiedenen Reaktivitäten im Analysenmaterial. Ferner haben sie den Nachteil, dass sie es praktisch nicht ermöglichen, zu bestimmen, ob eine Treponemainfektion noch akut oder bereits abgeklungen ist. Dies beruht unter anderem darauf, dass Antikörper gegen Treponemaantigen noch sehr lange nachweisbar sind, selbst wenn die eigentliche Infektion - das heißt das Vorliegen von Erregern - bereits abgeklungen ist. Ferner sind die Tests aus dem Stand der Technik teilweise umständlich von der Verfahrensführung und somit für den Routinebereich und Hochdurchsatztechnologien ungeeignet.

Der vorliegenden Erfindung lag das technische Problem zugrunde, Mittel und Verfahren anzugeben, die einerseits möglichst einfach zu handhaben sind und ferner eine serologische Verlaufskontrolle des tatsächlichen Infektionsgrads ermöglichen.

Das vorliegende Problem wird gelöst durch einen Träger für die Diagnostik und/oder Verlaufskontrolle einer Treponemainfektion, der mindestens ein immobilisiertes Cardiolipin und mindestens ein immobilisiertes Treponema-spezifisches Antigen enthält.

Bei einer weiteren bevorzugten Ausführungsform wird das Cardiolipin zusammen mit Lecithin und Cholesterin eingesetzt. Diese Kombination aus Cardiolipin, Lecithin und Cholesterin wird im Folgenden auch als VDRL-Antigen bezeichnet.

Bei einer weiteren bevorzugten Ausführungsform umfasst das VDRL-Antigen die genannten Bestandteile in folgenden Massenverhältnissen Cardiolipin : Lectihin : Cholesterin in den Verhältnissen 0,1 bis 4,0: 1 bis 5,0: 1 bis 10,0. Bei einer weiteren bevorzugten Ausführungsform sind die Massenverhältnisse 1 bis 3,0 für Cardiolipin : 1 bis 3 für Lecithin : 5 bis 10 für Cholesterin.

Bei einer weiteren bevorzugten Ausführungsform enthält der Träger mehrere Positionen mit Cardiolipin, mindestens zwei, vorzugsweise mindestens drei, besonders bevorzugt mindestens vier Positionen, an denen das Cardiolipin in verschiedenen Konzentrationen vorliegt. Diese Konzentrationen sind für die einzelnen Positionen vorzugsweise wie folgt: 0,10 bis 1,00 mg/ml (Position 1), 0,05 - 0,50 mg/ml (Position 2), 0,02 bis 0,2 mg/ml (Position 3), 0,01 bis 0,1 mg/ml (Position 4).

Bei einer weiteren bevorzugten Ausführungsform enthält der Träger mehrere Positionen mit VDRL-Antigen, mindestens zwei, vorzugsweise mindestens drei, besonders bevorzugt mindestens vier Positionen, an denen das VDRL-Antigen in verschiedenen Konzentrationen vorliegt.

Bei einer weiteren bevorzugten Ausführungsform enthält der Träger mindestens zwei, vorzugsweise mindestens drei, besonders bevorzugt mindestens vier verschiedene Treponema-spezifische Antigene, wobei jedes Antigen in einer unterschiedlichen Position auf dem Träger immobilisiert ist.

Bei einer weiteren bevorzugten Ausführungsform werden die Treponema-spezifischen Antigene ausgewählt aus Antigenen von Treponema pallidum, vorzugsweise handelt es sich hierbei um die 15 kD, 17 kD, 44,5 kD und 47 kD Antigene.

Bei einer weiteren bevorzugten Ausführungsform enthält der Träger weitere Kontrollen, die geeignet sind, die sachgemäße Durchführung des erfindungsgemäßen Verfahrens anzuzeigen. Zu diesen Kontrollen zählt bspw. eine Serumkontrolle, vorzugsweise Protein A. Diese Kontrolle zeigt an, dass der Teststreifen in der Tat beim Durchführen des Verfahrens mit Serum in Kontakt gebracht worden ist.

Bei einer weiteren bevorzugten Ausführungsform stellt die Kontrolle eine sog. Cut-Off-Kontrolle dar, welche bspw. eine Verdünnung von gereinigtem humanem Immunoglobulin enthält. Diese Kontrolle dient der Auswertung, indem ein schwächeres Signal als die Cut-off-Kontrolle einem negativen Testergebnis entspricht, während ein stärkeres Signal einem positiven Testergebnis entspricht. Als Trägermaterialien kommen Nitrocellulose, PVDF (Polyvinylidendifluorid), Nylon, Celluloseacetat, Polystyrol in Betracht, wobei Nitrocellulose besonders bevorzugt ist. Es hat sich gezeigt, dass beim Einsatz von Nitrocellulose ein besonders gutes Signal- Hintergrundverhältnis, insbesondere für das VDRL-Signal erhalten wird.

Bei einer weiteren bevorzugten Ausführungsform stellen Kontrollen sogenannte Konjugat-Kontrollen dar. Diese Kontrollen zeigen an, dass der Teststreifen in der Durchführung mit dem entsprechenden Konjugat, welches gegen humanes IgG bzw. humanes IgM bzw. humanes IgA gerichtet ist, in Kontakt gebracht worden ist.

Bei einer weiteren bevorzugten Ausführungsform ist der Träger als Teststreifen ausgestaltet, insbesondere in einem Format, das den Einsatz in herkömmlichen Verfahren der Immundiagnostik erlaubt

Bei einer weiteren bevorzugten Ausführungsform ist der Träger als Westernblot ausgestaltet. Dabei enthält der Träger die diversen Reagenzien in immobilisierter Form.

Das oben genannte technische Problem wird ebenfalls gelöst durch ein Verfahren zur Diagnostik und/oder Verlaufskontrolle einer Treponemainfektion, das dadurch gekennzeichnet ist, dass ein oben genannter Träger mit einer Patientenprobe in Kontakt gebracht wird und so das Vorliegen von Antikörpern gegen ein Treponema-Antigen und/oder Cardiolipin in der Patientenprobe bestimmt wird.

Vorzugsweise handelt es sich bei der Patientenprobe um eine Blut-, Serum-, Plasma-, Liquor- oder Synovialflüssigkeitsprobe des zu untersuchenden Patienten. Der Verfahrensablauf entspricht den üblichen Verfahren auf dem Gebiet der Immundiagnostik und ist dem Fachmann geläufig.

Bei einer weiteren bevorzugten Ausführungsform werden die Träger nach der Testdurchführung mit der Auswertesoftware ViraScan® (automatisch) ausgelesen und ausgewertet. Die Träger werden mit ViraScan® densitometrisch analysiert. Dabei werden die Intensitäten der VDRL-IgG- und VDRL-IgM-Banden und die Treponemen-spezifischen-Antigenbanden mit der Cut-off-Kontrollbandenintensität, die sich ebenfalls auf den Trägern befindet, verglichen. Dies erlaubt eine quantitative Bestimmung der Antikörperreaktivitäten.

Das oben genannte technische Problem wird ferner gelöst durch einen Testkit zur Diagnose einer Treponemainfektion und/oder der Verlaufskontrolle einer Treponemainfektion, wobei der Testkit einen eingangs genannten Träger enthält sowie die weiteren Reagenzien zur Durchführung des diagnostischen Verfahrens sowie eine Gebrauchsanleitung zur Durchführung des Assays.

Mit dem neuen Testverfahren bzw. Träger können in einem Ansatz sowohl Treponema pallidum spezifische Antikörper jeweils gegen das 47 kD -, das 44,5 kD -, das 17 kD und das 15 kD Protein als auch Antikörper gegen das VDRL Antigen (Cardiolipin : Lecithin : Cholesterin), insbesondere Cardiolipin, nachgewiesen werden.

Durch die Kombination von Treponema pallidum spezifischen Antigenen (47 kD -, 44,5 kD-, 17 kD-, 15 kD - Proteine) mit nicht-Treponema pallidum Antigenen (z.B. Cardiolipin) in einem Testformat kann gewährleistet werden, dass eine sicherere, besser automatisierbare und aussagekräftigere Syphilis-Diagnostik erfolgen kann.

Im Rahmen der Stufendiagnostik zur Diagnose der Syphilis ist es somit möglich, nach einem Screening Test (TPPA, TPHA, TPLA, Tp Elisa) anschließend mit dem erfindungsgemäßen Träger bzw. Verfahren sowohl die serologische Bestätigungsreaktion, als auch die Analyse zur Feststellung der Behandlungsbedürftigkeit, als auch die serologische Verlaufskontrolle durchführen zu können.

Insbesondere die Verlaufskontrolle einer Treponemainfektion wird mittels des erfindungsgemäßen Trägers zuverlässig und einfach möglich. Das mit VDRL-Antigen erhaltene Meßsignal ist eine zuverlässige Größe für den Grad der Treponemainfektion. Während die in der Patientenprobe vorliegende Reaktivität mit den Treponema-Antigenen relativ unabhängig ist von dem Grad der Infektion (d.h. der Schwere der Infektion) gibt das VDRL-Signal hierzu aussagekräftigere Werte.

Bei der Auswertung der VDRL-Signale werden die Intensitäten der einzelnen VDRL-Banden mit der Intensität der Cut-off Kontrollbande verglichen und mit 0, 1 oder 2 ViraBlot Einheiten bewertet, je nachdem ob die VDRL-Banden schwächer, gleich stark oder eindeutig stärker als die Cut-off Kontrollbande erscheinen. Die Summe der einzelnen VDRL-ViraBlot Einheiten gibt die Reaktivität der vorhandenen Lipoidantikörper an.

Hierbei korreliert die Zunahme der Summe der VDRL-ViraBlot Einheiten mit einer Zunahme der Reaktivität der Lipoidantikörper. Eine ViraBlot-Einheit bedeutet dabei, dass der Wert 1 vorgegeben wird, wann ein Signal, das mit einer VDRL-Bande beobachtet wird, die gleiche Intensität aufweist wie das Signal, das mit Cut-off Kontrollbande erhalten wird. 0 bedeutet dabei, dass das Signal der VDRL-Bande deutlich schwächer ist als das Signal der Cut-off Kontrollbande und der Wert 2 bedeutet, dass die Intensität der VDRL-Bande deutlich höher ist, als die Intensität der Cut-off Kontrollbande. Je höher die Anzahl der so ermittelten ViraBlot-Einheiten ist, um so größer ist die Reaktivität von Antikörpern in der Analysenprobe mit den VDRL-Banden.

Schließlich erlaubt der erfindungsgemäße Träger und das damit ausgeführte Verfahren eine Unterscheidung zwischen den Antikörperklassen IgG und IgM, die gegen VDRL gerichtet sind. Dies hat insbesondere auch Vorteile bei der Diagnose, z.B. bei der Beurteilung der Behandlungsbedürftigkeit von Kindern mit fraglicher konnataler Syphilis.

Es war insbesondere überraschend, dass es gelungen ist, die körpereigenen Lipide (Cardiolipin, Lecithin und Cholesterin) derart auf einen festen Träger zu immobilisieren, dass deren Reaktivität mit den Antikörpern des Patientenmaterials erhalten bleibt. Dies war keineswegs zu erwarten, da es sich bei den Lipidstrukturen, insbesondere beim Cardiolipin, um relativ labile dreidimensionale Strukturen handelt, so dass das Aufbringen auf einen festen Träger ein hohes Risiko der Zerstörung der relevanten Epitope darstellte. Überraschenderweise bleiben die diagnostisch relevanten Epitope jedoch auch nach Fixierung auf dem festen Träger erhalten und erlauben somit einen raschen, einfachen und dennoch sicheren Nachweis der gegen Cardiolipin gerichteten Antikörper.

### Kurze Beschreibung der Abbildungen

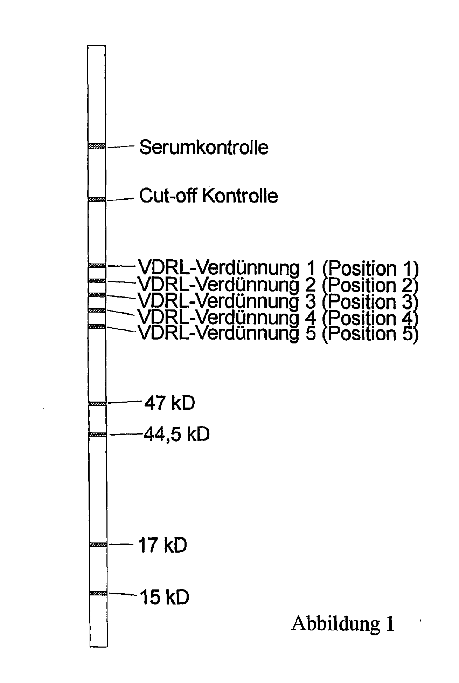

**Abbildung 1** zeigt einen erfindungsgemäßen Träger mit VDRL-Antigenen verschiedener Konzentrationen sowie mit vier verschiedenen Treponema pallidum Antigenen, Serumkontrolle und Cut-off Kontrolle
**Abbildung 2** zeigt erfindungsgemäßige Träger für die VDRL-IgG-Verlaufskontrolle bei einem Patienten mit Lues II
**Abbildung 3** zeigt die Korrelation von VDRL VraBlot Einheiten mit den bekannten, herkömmlich ermittelten, VDRL- oder Cardiolipin-KBR Titer
**Abbildung 4** zeigt IgG und IgM- Antworten der erfindungsgemäßen Träger nach einer Reaktion mit menschlichem Serum bei einem Lues II Patienten mit HIV Infektion
**Abbildung 5** zeigt IgG und IgM- Antworten der erfindungsgemäßen Träger nach einer Reaktion mit menschlichem Serum bei einer Mutter und ihrem Kind mit fraglicher konnataler Syphilis

### Die folgenden Beispiele erläutern weiterhin die Erfindung.

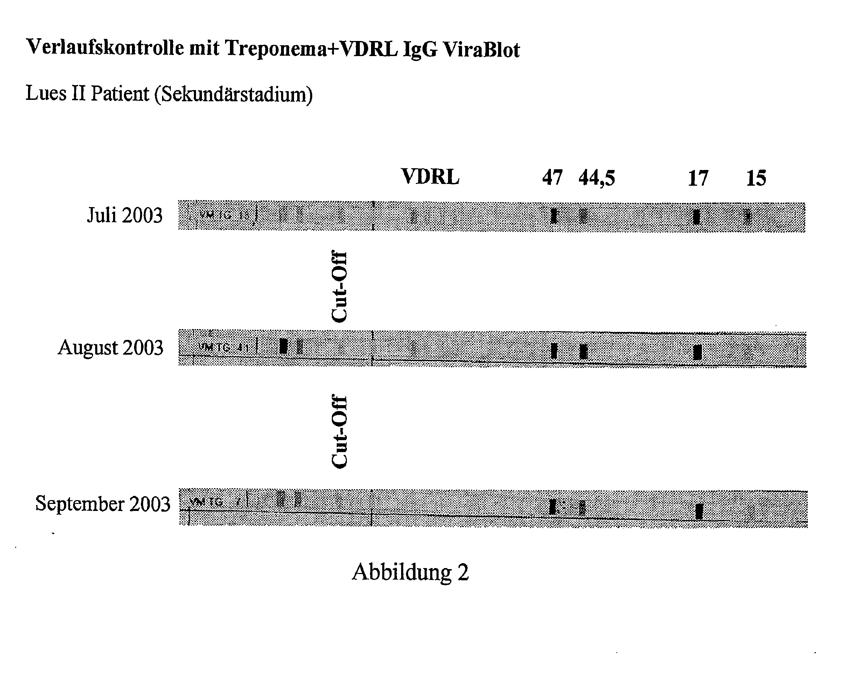

Abbildung 2 zeigt Teststreifen vom Typ IgG zur VDRL-Antigen-Verlaufskontrolle bei einem Lues II Patienten. Die Tests wurden im Abstand von je einem Monat, in einem Zeitraum von 3 Monaten durchgeführt. Die Treponema spezifischen Antigenbanden sind auf jedem der Teststreifen deutlich erkennbar. Bei den VDRL-Antigenbanden, ist der Abfall des VDRL-IgG Titers im Verlauf der abklingende Infektion nach Therapie gut zu sehen.

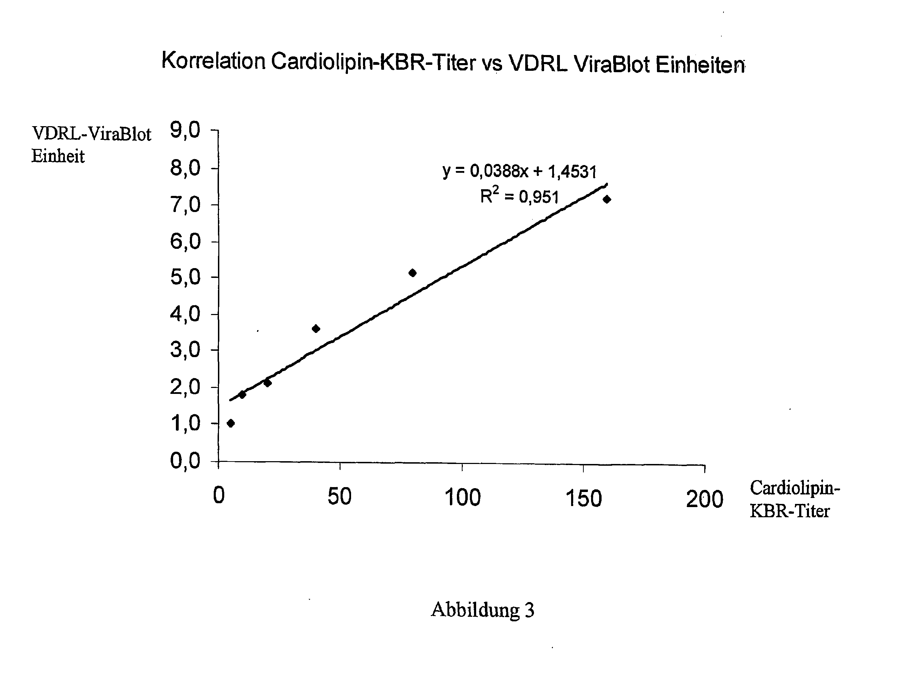

Die VDRL ViraBlot Einheiten korrelieren mit den bekannten, herkömmlich ermittelten, VDRL oder Cardiolipin-KBR Titern. Diese Korrelation wird im Folgenden in Abbildung 3 dargestellt.

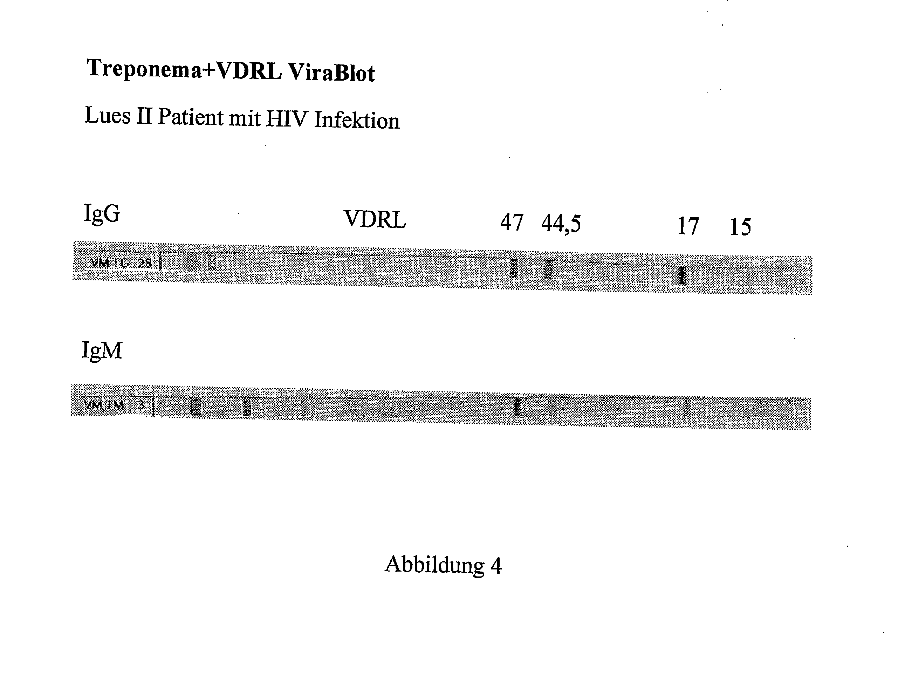

In Abbildung 4 sind Treponema+VDRL ViraBlot IgG und IgM-Immunoantworten zu Lues II bei einem HIV Patienten dargestellt. Die Banden der Treponemen spezifischen Antigene 47 kD, 44,5 kD, 17 kD und 15 kD sind jeweils im IgG und IgM deutlich erkennbar. VDRL-Banden sind nur im IgM und nicht im IgG deutlich sichtbar.

Summiert man die einzelnen ViraBlot-Einheiten für die VDRL-ViraBlot IgG- und IgM- Antikörperbefunde auf und wertet diese mit den Treponema spezifischen Banden aus, so sieht man, dass der Patient, je nach Klinik, entweder kürzlich einer Infektion unterlag oder sich noch in einer aktiven Syphilis-Phase befindet. Das Aufbringen von VDRL-Antigenen und Treponema spezifischen Antigenen auf einem Träger, vereinfacht die Testdurchführung und lässt eine schnelle serodiagnostische Aussage auf einem Blick zu. Anstatt wie bisher zwei Tests hintereinander durchzuführen (z.B. VDRL und 19S-IgM-FTA-ABS), sind diese nun in einem Schritt integriert.

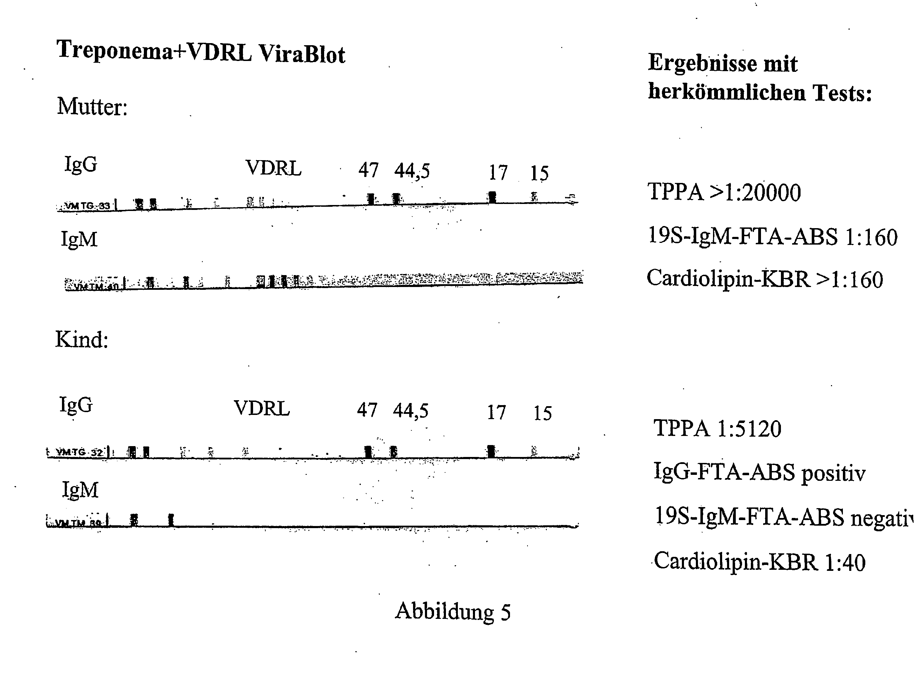

In Abbildung 5 sind die Immunoreaktivitäten von VDRL und Treponema spezifischen Antigenen im IgG und IgM Imunoassay bei einer Mutter und ihrem Kind mit Verdacht auf konnatale Syphilis dargestellt. Im IgG bei Mutter und Kind und im IgM bei der Mutter sind jeweils die Banden für die Antigene 47 kD, 44,5 kD, 17 kD und 15 kD deutlich erkennbar. Die Summe der einzelnen ViraBlot-Einheiten für die VDRL-ViraBlot IgG- und IgM- Antikörperbefunde bei der Immunantwort der Mutter zeigen eine eindeutige Reaktion für die Lipoidantikörper. Treponema spezifische IgG und IgM Banden, zusammen mit den VDRL- ViraBlot IgG- und IgM- Antikörperbefunden zeigen somit an, dass die Mutter erst kürzlich einer Treponema-Infektion unterlag, oder je nach Anamnese, sich noch in einer aktiven Infektionsphase befindet.

Da IgM-Antikörper in der Regel nicht diaplazentar auf den Fetus übertragen werden, würde eine Therapiebedürftigkeit erst bestehen, wenn beim Neugeborenen Treponema spezifische IgM- Antikörper und/oder IgM-VDRL-Antikörper auf eine Infektion hindeuten, was in diesem Beispiel nicht der Fall ist. Daher lässt der Nachweis von IgG-Antikörpern beim Kind in diesem Falle darauf schließen, dass sie diaplazentar von der Mutter übertragen worden sind.

Im Gegensatz zu den herkömmlichen Tests unterscheidet die hier vorliegende Erfindung zwischen anti-VDRL-IgG- und anti-VDRL-IgM-Antikörpern. Diese Differenzierung ermöglicht eine bessere Aussage der Behandlungsbedürftigkeit bei einem Kind mit fraglicher konnataler Syphilis.

Nach den herkömmlichen Testverfahren und den daraus resultierenden Ergebnissen (TPPA, 19S-IGM-FTA-ABS, IgG-FTA-ABS und Cardiolipin-KBR) wäre das Kind in diesem Beispiel therapiert worden, insbesondere da der Cardiolipin-KBR Wert positiv ausfällt (1:40).

Mit der hier vorliegenden Erfindung ergibt sich in diesem Beispiel sowohl für die IgM-VDRL-Antikörper als auch für die Treponema spezifischen IgM-Antikörper ein negatives Testergebnis, das nicht auf eine Therapiebedürftigkeit des Kindes hinweist. Dieses Beispiel belegt den Vorteil eines separaten Nachweises verschiedener Antikörperreaktivitäten in dem Analysenmaterial.

### Herstellung des Treponema Testsstreifens

### Herstellung des VDRL Antigens

VDRL Antigen wurde aus einer Mischung aus den Einzelkomponenten Cardiolipin, Lecithin und Cholesterin in Ethanol hergestellt. Cardiolipin wurde aus Rinderherz gereinigt (Avanti Polar Lipids Inc., Alabaster, AL, USA), Lecithin aus Hühnerei (Avanti Polar Lipids Inc., Alabaster, AL, USA) und Cholesterin aus Wollfett (Avanti Polar Lipids Inc., Alabaster, AL, USA). Die Einzelkomponenten wurden in Reinheitsstufen von mindestens 98 % eingesetzt. Die Einzelkomponenten lagen jeweils als Trockensubstanz vor und wurden in Ethanol (100%) aufgenommen. Die Konzentration von Cardiolipin betrug 4 g/l in Ethanol (100%), die Konzentration von Lecithin betrug 100 g/l in Ethanol (100%) und die Konzentration von Cholesterin betrug 25g/l in Ethanol (100 %). In der Reihenfolge Cardiolipin, Lecithin und Cholesterin wurden die Einzelkomponenten entsprechend dem gewünschten Mischungsverhältnis gemischt. Das Gemisch wird bei Raumtemperatur 12-24 Stunden im Dunkeln vor einer weiteren Verarbeitung aufbewahrt. In der VDRL Mischung lagen die Massenverhältnisse von Cardiolipin:Lecithin:Cholesterin im Bereich von (0,1-4,0) : (0,1-5,0) : (0,1-10,0). Gute Ergebnisse wurden mit einer Mischung aus Cardiolipin:Lecithin:Cholesterin mit einem Massenverhältnis von 2,0:1,4:9,0 erzielt.

### Herstellung von VDRL - Verdünnungen

VDRL Antigen wurde in phosphatgepufferter Kochsalzlösung pH 7,2 stufenweise in 1,4-bis 1000 - fachen Verdünnungen hergestellt.

### Herstellung von Treponema spezifischen Antigenlösungen

47 kD Protein aus Treponema pallidum (Capricorn, USA), 44,5 kD Protein aus Treponema pallidum (Lee Laboratories, USA), 17 kD Protein aus Treponema pallidum (Lee Laboratories, USA) und 15 kD Protein aus Treponema pallidum (Lee Laboratories, USA) wurden zur Herstellung der Treponema spezifischen Antigenlösungen verwendet. Die Verdünnungen der Antigene erfolgte in phosphatgepufferter Kochsalzlösung pH 7,2. Es wurden Konzentrationen zwischen 1 µg/ml und 200 µg/ml pro Antigen verwendet Gut geeignet haben sich beim 47 kD Protein Lösungen mit einer Konzentration von 60 µg/ml, beim 44,5 kD Protein Lösungen mit einer Konzentration von 5 µg/ml, beim 17 kD Protein Lösungen mit einer Konzentration von 10 µg/ml und beim 15 kD Protein Lösungen mit einer Konzentration von 20 µg/ml.

### Immobilisierung der Antigene auf festphasigem Trägermaterial

Als festphasiges Trägermaterial wurde Nitrocelluose (Schleicher und Schüll, Dassel, Deutschland) eingesetzt. Die entsprechend verdünnten Antigene (Konzentrationen: VDRL: 1,40 - fache, 28,6 - fache, 55,6 - fache und 111,1 - fache Verdünnung der VDRL Antigenlösung; 47 kD - Protein: 60 µg/ml; 44,5 kD - Protein: 5 µg/ml; 17 kD - Protein: 10 µg/ml; 15 kD - Protein: 20 µg/ml) konnten durch passive Adsorption auf Nitrocellulose als festphasigem Trägermaterial gebunden werden. Die Antigen-Lösungen wurden mit einem Dispensierautomaten mit Tropfengrößen von 10,67 nl bis 85 nl und Flussraten von 0,5 µl/cm bis 5,0 µl/cm aufgetragen.

### Blockierung freier Bindestellen

Mit Antigen beschichtete Nitrocellulose wurde mit einem Puffer aus Trishydroxymethylaminomethan (3,25 g/l), Natriumchlorid (7,51 g/l), Tween 20 (3,83 ml/l), Thimerosal (0,02 g/l), Milchpulver (40 g/l), pH 7,5, bei 37 °C 30 min inkubiert, anschließend in einem Puffer aus Trishydroxymethylaminomethan (3,25 g/l), Natriumchlorid (7,51 g/l), Tween 20 (3,83 ml/l), Thimerosal (0,02 g/l), Milchpulver (5 g/l), pH 7,5, bei 37 °C 30 min inkubiert und getrocknet.
Zur weiteren Verwendung wurde die blockierte Nitrocellulose in Streifen geschnitten (Teststreifen).

### Entwicklung der Nitrocellulosestreifen mit Probenmaterial

### Als Probenflüssigkeit eignen sich Serum, Plasma, CSF (zerebrospinale Flüssigkeit) oder Synovialflüssigkeit.

Der mit Antigen beschichtete Nitrocellulosestreifen (=Teststreifen, zum Beispiel Darstellung 1) wird in 1,5 ml Waschpuffer bestehend aus Trishydroxymethylaminomethan (3,25 g/l), Natriumchlorid (7,51 g/l), Tween 20 (3,83 ml/l), Thimerosal (0,02 g/l), Milchpulver (5 g/l), pH 7,5, bei Raumtemperatur 5 min auf einem Wippschüttler in einer Wanne inkubiert. Anschließend wird der Puffer dekantiert. 20 µl Probenflüssigkeit werden zusammen mit 1,5 ml Waschpuffer auf den Teststreifen gegeben. Der Teststreifen wird 30 min auf dem Wippschüttler inkubiert. Nach der Inkubation mit Probenflüssigkeit wird der Teststreifen dreimal mit jeweils 1,5 ml Waschpuffer gewaschen und anschließend mit anti-human-IgG oder anti-human-IgM oder anti-human-IgA - alkalische Phosphatase-Konjugat inkubiert. Anschließend wird mit jeweils 1,5 ml Waschpuffer dreimal jeweils 5 min gewaschen. In einem weiteren Waschschritt wird mit destilliertem Wasser für 1 min nachgewaschen und anschließend mit Chromogen/Substrat Lösung (BCIP/NBT) entwikkelt. Die Entwicklung wird entsprechend der Färbung einer Cut-Off Kontrolle durch Dekantieren der Entwicklungslösung und dreimaliges Waschen mit jeweils 1,5 ml destilliertem Wasser gestoppt.

### Interpretation der Färbungen auf den Teststreifen

Auf den Teststreifen werden Färbeintensitäten der Antigenlinien mit der Färbeintensität der Cut-Off Kontrolle verglichen. Eine Färbeintensität kann durch Scannen (handelsübliche Farbscanner; Programm ViraScan®, Viramed, Planegg, Deutschland) der entwikkelten Teststreifen quantifiziert werden. Aus den Messwerten der Färbeintensitäten werden Verhältnisse zur Cut-Off Kontrolle berechnet (Ratio). Ist das Ratio größer oder gleich eins, so wird eine entsprechend gefärbte Antigenlinie als positiv gewertet. Ist das Ratio kleiner als eins aber größer oder gleich 0,5 so wird die entsprechende Antigenlinie grenzwertig gewertet. Ist das Ratio kleiner als 0,5 so wird die entsprechende Antigenlinie negativ gewertet.

Eine oder mehrere positive Antigenlinien der Proteine 47 kD, 44,5 kD, 17 kD oder 15 kD weisen auf das Vorhandensein entsprechender Antikörper in der jeweiligen untersuchten Probe hin.

Eine oder mehrere positive Antigenlinien der aufgetragenen VDRL Verdünnungsstufen weisen auf das Vorhandensein von anti-Cardiolipin-Antikörpern eines entsprechenden Titers hin. Es besteht eine Korrelation zwischen dem VDRL-Tter, ermittelt nach dem herkömmlichen VDRL Testverfahren und der quantifizierbaren Färbeintensität der VDRL Antigenlinien auf dem Teststreifen (Abbildung 3). Somit ist das Trägergebundene VDRL ein geeignetes Mittel zum quantitativen, zumindest semi-quantitativen, Nachweis von Anti-Cardiolipin-Antikörpem und kann somit zur Verlaufskontrolle einer Treponemainfektion herangezogen werden.

## Patentansprüche

1. Träger für die Diagnostik und/oder Verlaufskontrolle einer Treponemainfektion, umfassend
a) mindestens ein immobilisiertes Cardiolipin und
b) mindestens ein immobilisiertes Treponema-spezifisches-Antigen.

2. Träger nach Anspruch 1, **dadurch gekennzeichnet, dass** das Cardiolipin zusammen mit Lecithin und Cholesterin als VDRL-Antigen vorliegt, wobei diese Produkte vorzugsweise in einem Massenverhältnis Cardiolipin : Lecithin : Cholesterin von 0,1 - 4,0 : 1 - 5,0 : 1-10 vorliegen.

3. Träger nach einem der Ansprüche 1 oder 2, **dadurch gekennzeichnet, dass** das Cardiolipin in mindestens zwei, vorzugsweise in mindestens drei, besonders bevorzugt in mindestens vier verschiedenen Konzentrationen an unterschiedlichen Positionen des Trägers vorliegt.

4. Träger nach einem der Ansprüche 1 bis 3, **dadurch gekennzeichnet, dass** mindestens zwei, vorzugsweise mindestens drei, besonders bevorzugt mindestens vier verschiedene Treponema-Antigene in unterschiedlichen Positionen auf dem Träger vorliegen.

5. Träger nach einem Ansprüche 1 bis 4, **dadurch gekennzeichnet, dass** die Antigene ausgewählt werden aus Treponema pallidum spezifischen Antigen, vorzugsweise dem 15kD, 17 kD, 44,5 kD und 47 kD Antigenen.

6. Träger nach einem der Ansprüche 1 bis 5, **dadurch gekennzeichnet, dass** der Träger weitere Kontrollen umfaßt.

7. Träger nach einem der Ansprüche 1 bis 6, **dadurch gekennzeichnet, dass** eine Kontrolle eine Serumkontrolle ist, vorzugsweise Protein A.

8. Träger nach einem der Ansprüche 1 bis 6, **dadurch gekennzeichnet, dass** eine Kontrolle eine Cut-off-Kontrolle ist, vorzugsweise enthaltend gereinigtes humanes Immunglobulin.

9. Träger nach einem der Ansprüche 1 bis 5, **dadurch gekennzeichnet, dass** er eine Serumkontrolle, welche vorzugsweise Protein A enthält und eine Cut-off-Kontrolle, welche vorzugsweise humanes Immunglobulin enthält, umfasst.

10. Träger nach einem der Ansprüche 1 bis 9, **dadurch gekennzeichnet, dass** der Träger ausgewählt wird aus Nitrocellulose, PVDF (Polyvinylidendifluorid), Nylon, Celluloseacetat, Polystyrol.

11. Träger nach einem der Ansprüche 1 bis 10, **dadurch gekennzeichnet, dass** der Träger als Teststreifen zum Einsatz in der Immundiagnostik ausgestaltet ist.

12. Träger nach einem der Ansprüche 1 bis 11, **dadurch gekennzeichnet, dass** der Träger als Immunoblot ausgestaltet ist.

13. Träger nach einem der Ansprüche 1 bis 12, **dadurch gekennzeichnet, dass** die auf den Träger aufgetragenen VDRL-Antigenbanden nach Reaktion mit einer Patientenprobe, vorzugsweise ausgewählt aus Blut, Serum, Plasma, Liquor oder Synovialflüssigkeit, eine Differenzierung zwischen anti-VDRL-IgG- und anti-VDRL-IgM Antikörpern, ermöglichen.

14. Verfahren zur Diagnostik und/oder Verlaufskontrolle einer Treponemainfektion, **dadurch gekennzeichnet, dass** ein Träger nach einem der Ansprüche 1 bis 13 mit einer Patientenprobe in Kontakt gebracht wird und das Vorliegen von Antikörpern gegen ein Treponema-Antigen und/oder ein Cardiolipin bestimmt wird.

15. Verfahren nach Anspruch 14, **dadurch gekennzeichnet, dass** die Reaktivität von Antikörpern aus dem Patientenserum mit dem Cardiolipin des Teststreifens mehrmals über einen längeren Zeitraum ermittelt wird.

16. Verfahren nach einem der Ansprüche 14 oder 15, **dadurch gekennzeichnet, dass** die Patientenprobe Blut, Serum, Plasma, Liquor oder Synovialflüssigkeit ist

17. Verfahren nach einem der Ansprüche 14-16, **dadurch gekennzeichnet, dass** die Auswertung mittels der Auswertesoftware ViraScan® erfolgt.

18. Verfahren nach einem der Ansprüche 14 bis 17, **dadurch gekennzeichnet, dass** anti-VDRL-IgG- und anti-VDRL-IgM Antikörper in einer Patientenprobe differenziert werden.

19. Testkit zur Diagnose einer Treponemainfektion und/oder der Verlaufskontrolle einer Treponemainfektion, enthaltend einen Träger nach einem der Ansprüche 1 bis 13 und weitere Reagenzien sowie eine Gebrauchsanleitung zur Durchführung des Nachweisverfahrens.

20. Verwendung eines Trägers nach einem der Ansprüche 1 bis 13 in der *in vitro* Diagnostik und/oder *in vitro* Verlaufskontrolle einer Treponemainfektion.

## Claims

1. A carrier for diagnostics and/or follow-up of a Treponema infection, comprising
a) at least one immobilized cardiolipin and
b) at least one immobilized Treponema-specific antigen.

2. The carrier according to claim 1, **characterized in that** the cardiolipin is present together with lecithin and cholesterol as VDRL antigen, said products being preferably present in a mass ratio of cardiolipin : lecithin : cholesterol of 0.1-4.0 : 1-5.0 : 1-10.

3. The carrier according to any one of claims 1 or 2, **characterized in that** the cardiolipin is present in at least two, preferably at least three, particularly preferably at least four different concentrations at different positions of the carrier.

4. The carrier according to any one of claims 1 to 3, **characterized in that** at least two, preferably at least three, particularly preferably at least four different Treponema antigens are present in different positions on the carrier.

5. The carrier according to any one of claims 1 to 4, **characterized in that** the antigens are selected from Treponema pallidum-specific antigen, preferably the 15kD, 17 kD, 44.5 kD and 47 kD antigen.

6. The carrier according to any one of claims 1 to 5, **characterized in that** the carrier comprises further controls.

7. The carrier according to any one of claims 1 to 6, **characterized in that** one control is a serum control, preferably protein A.

8. The carrier according to any one of claims 1 to 6, **characterized in that** one control is a cut-off control, preferably comprising purified human immunoglobulin.

9. The carrier according to any one of claims 1 to 5, **characterized in that** it comprises a serum control which preferably comprises protein A and a cut-off control which preferably comprises human immunoglobulin.

10. The carrier according to any one of claims 1 to 9, **characterized in that** the carrier is selected from nitrocellulose, PVDF (polyvinylidene difluoride), nylon, cellulose acetate, polystyrene.

11. The carrier according to any one of claims 1 to 10, **characterized in that** the carrier is designed as a test strip for use in immunodiagnostics.

12. The carrier according to any one of claims 1 to 11, **characterized in that** the carrier is designed as an immunoblot.

13. The carrier according to any one of claims 1 to 12, **characterized in that** the VDRL antigen bands applied to the carrier allow a differentiation between anti-VDRL-IgG and anti-VDRL-IgM antibodies after reaction with a patient's sample, preferably selected from blood, serum, plasma, liquor or synovial fluid.

14. A method for diagnostics and/or follow-up of a Treponema infection, **characterized in that** a carrier according to any one of claims 1 to 13 is contacted with a patient's sample and the presence of antibodies against a Treponema antigen and/or a cardiolipin is determined.

15. The method according to claim 14, **characterized in that** the reactivity of antibodies from a patient's serum with the cardiolipin of the test strip is determined several times over a prolonged period of time.

16. The method according to any one of claims 14 or 15, **characterized in that** the patient's sample is blood, serum, plasma, liquor or synovial fluid.

17. The method according to any one of claims 14 to16, **characterized in that** the assessment is performed through the evaluation software ViraScan®.

18. The method according to any one of claims 14 to 17, **characterized in that** anti-VDRL-IgG and anti-VDRL-IgM antibodies are differentiated in a patient's sample.

19. A test kit for the diagnosis of a Treponema infection and/or the follow-up of a Treponema infection, comprising a carrier according to any one of claims 1 to 13 and further reagents as well as an instruction manual for carrying out the detection method.

20. Use of a carrier according to any one of claims 1 to 13 in *in-vitro* diagnostics and/or *in-vitro* follow-up of a Treponema infection.

## Revendications

1. Support pour le diagnostic et/ou le contrôle de l'évolution d'une infection à tréponème, comprenant
a) au moins une cardiolipine immobilisée et
b) au moins un antigène spécifique du tréponème immobilisé.

2. Support selon la revendication 1, **caractérisé en ce que** la cardiolipine, conjointement à de la lécithine et de la cholestérine, est présente en tant qu'antigène VDRL, ces produits se présentant de préférence dans un rapport en poids de cardiolipine : lécithine : cholestérine de 0,1 à 4,0 : 1 à 5,0 : 1 à 10.

3. Support selon la revendication 1 ou 2, **caractérisé en ce que** la cardiolipine se présente en au moins deux, de préférence au moins trois, due manière particulièrement préférée au moins quatre concentrations différentes en des positions différentes du support.

4. Support selon l'une des revendications 1 à 3, **caractérisé en ce qu'**au moins deux, de préférence au moins trois, de manière particulièrement préférée au moins quatre antigènes de tréponème différents sont présents en différentes positions sur le support.

5. Support selon l'une des revendications 1 à 4, **caractérisé en ce** les antigènes sont choisis parmi les antigènes spécifiques de *Treponema pallidum,* de préférence parmi les antigènes de 15 kD, 17 kD, 44,5 kD et 47 kD.

6. Support selon l'une des revendications 1 à 5, **caractérisé en ce qu'**il comprend d'autres moyens de contrôle.

7. Support selon l'une des revendications 1 à 6, **caractérisé en ce qu'**un moyen de contrôle est un moyen de contrôle sérique, de préférence la protéine A.

8. Support selon l'une des revendications 1 à 6, **caractérisé en ce qu'**un moyen de contrôle est un moyen de contrôle de seuil de coupure, de préférence contenant une immunoglobuline humaine purifiée.

9. Support selon l'une des revendications 1 à 5, **caractérisé en ce qu'**il comprend un moyen de contrôle sérique qui contient de préférence la protéine A et un moyen de contrôle de seuil de coupure qui contient de préférence une immunoglobuline humaine.

10. Support selon l'une des revendications 1 à 9, **caractérisé en ce qu'**il est choisi parmi la nitrocellulose, le PVDF (polydifluorure de vinylidène), le Nylon, l'acétate de cellulose, le polystyrène.

11. Support selon l'une des revendications 1 à 10, **caractérisé en ce qu'**il est conçu sous forme de bandelettes de test pour utilisation dans le diagnostic immun.

12. Support selon l'une des revendications 1 à 11, **caractérisé en ce qu'**il est conçu comme un immunoblot.

13. Support selon l'une des revendications 1 à 12, **caractérisé en ce que** les bandes d'antigènes VDRL appliquées sur le support après réaction avec un échantillon d'un patient, choisi de préférence parmi le sang, le sérum, le plasma, le liquide céphalo-rachidien ou le liquide synovial, permettent une différenciation entre les anticorps IgG anti-VDRL et IgM anti-VDRL.

14. Procédé de diagnostic et/ou de contrôle de l'évolution d'une infection à tréponème, **caractérisé en ce qu'**un support selon l'une des revendications 1 à 13 est mis en contact avec un échantillon d'un patient et la présence d'anticorps contre un antigène de tréponème et/ou une cardiolipine est déterminée.

15. Procédé selon la revendication 14, **caractérisé en ce que** la réactivité d'anticorps du sérum du patient avec la cardiolipine de la bandelette de test est déterminée à plusieurs reprises sur une durée prolongée.

16. Procédé selon la revendication 14 ou 15, **caractérisé en ce que** l'échantillon du patient est du sang, du sérum, du plasma, du liquide céphalo-rachidien ou du liquide synovial.

17. Procédé selon l'une des revendications 14 à 16, **caractérisé en ce que** l'évaluation est effectuée au moyen du logiciel d'analyse ViraScan®.

18. Procédé selon l'une des revendications 14 à 17, **caractérisé en ce que** les anticorps IgG anti-VDRL et les anticorps IgM anti-VDRL sont différenciés dans un échantillon d'un patient.

19. Kit de test pour le diagnostic d'une infection à tréponème et/ou le contrôle de l'évolution d'une infection à tréponème, contenant un support selon l'une des revendications 1 à 13 et d'autres réactifs ainsi qu'un mode d'emploi pour réaliser le procédé de détection.

20. Utilisation d'un support selon l'une des revendications 1 à 13 dans le diagnostic *in vitro* et/ou le contrôle d'évolution *in vitro* d'une infection à tréponème.
